# EUROPÄISCHE PATENTSCHRIFT

(11) **EP 0 204 781 B1**
(45) Veröffentlichungstag und Bekanntmachung des Hinweises auf die Patenterteilung: **26.04.1995**
(21) Anmeldenummer: 86900093.5
(22) Anmeldetag: 21.11.1985
(51) Int. Cl.: C07D 501/18, C07D 501/04, C07F 7/18

(54) **VERFAHREN ZUR HERSTELLUNG BISSILYLIERTER 3-IODMETHYLCEPHALOSPORINDERIVATEN**
PROCESS FOR THE PRODUCTION OF BISSILYLATED 3-IODOMETHYLCEPHALOSPORINE DERIVATIVES
PROCEDE POUR LA PREPARATION DE DERIVES DE 3-IODO-METHYLCEPHALOSPORINES BISSILYLES

(30) Priorität: 23.11.1984 AT 3715/84; 23.11.1984 AT 3716/84
(43) Veröffentlichungstag der Anmeldung: 17.12.1986
(60) Teilanmeldung: 94105662.4
(73) Patentinhaber: BIOCHEMIE Gesellschaft m.b.H., 6250 Kundl Tirol (AT)
(72) Erfinder: ASCHER, Gerd, A-6250 Kundl (AT); THALER, Heinrich, A-6322 Kirchbichl (AT); LUDESCHER, Johannes, A-6250 Breitenbach (AT)
(74) Vertreter: Kleine-Deters, Johannes
(86) Internationale Anmeldenummer: EP8500635
(87) Internationale Veröffentlichungsnummer: WO8603204

(56) Entgegenhaltungen:
- EP-A- 0 154 417
- Handbook of derivatives for chromatography, 1974, K.Blau/G.King, ed.Heyden&Son, pp. 179-180
- J. Antib., XLI (10), 1988, pp. 1374-1394, R. Lattrell et al.
- Reagents for organic synthesis, 1982, p. 1311, Fieser&Fieser, ed. J.Wiley&Sons, Inc.
- Methoden der Organischen Chemie, IX, 1960, Houben-Weyl

## Beschreibung

Cephalosporine sind wertvolle, in der Humanmedizin verwendete, Antibiotika. Ein Teil dieser Verbindungen (z. B. Cefalotin, Cefotaxim) leitet sich von dem Grundkörper 7-Aminocephalosporansäure (7-ACA) ab. 7-ACA kann durch Fermentation von Cephalosporin C, anschließende Isolierung des Naturprodukts und Seitenkettenabspaltung einfach hergestellt werden und ist ein leicht erhältliches Handelsprodukt.

Ein anderer, wesentlich größerer Teil der heute therapeutisch verwendeten Cephalosporine leitet sich hingegen von 7-Aminocephalosporansäuren ab, die einen anderen Rest als 7-ACA in 3-Stellung tragen (z. B. Cefaloridin, Cefamandol, Cefazolin, Cefoperazon, Ceftazidime, Ceftriaxon, Cefmenoxim, Cefonicid). Für die Herstellung von derartigen Verbindungen muß üblicherweise zunächst bei 7-ACA ein Austausch des Substituenten in 3-Stellung durchgeführt und die 3-substituierte 7-ACA isoliert werden. Ein weiterer Schritt führt dann zur Acylierung in 7-Stellung und zur Isolierung des gewünschten Cephalosporinantibiotikums.

Die Reaktion kann auch umgekehrt durchgeführt werden, das heißt, daß zuerst 7-ACA mit dem gewünschten Rest in 7-Stellung acyliert und anschließend das Acylderivat der 7-ACA isoliert wird. Nachfolgender Austausch in wäßrigen Puffersystemen führt dann üblicherweise zum gewünschten Cephalosporin-Endprodukt.

Beide Reaktionen haben Begrenzungen, das heißt, sie lassen sich nicht wahllos mit jeder Kombination von gewünschten Resten in 3- oder 7-Stellung durchführen. In allen Fällen muß über Acylierung/Austausch und/oder Isolierung im protischen Medium gearbeitet werden. Enthält die Seitenkette in 7-Stellung selbst eine acylierbare oder aktivierbare Funktion, so muß diese Gruppe zuerst mit einer ausgewählten Schutzgruppe geschützt und nach der Isolierung des gewünschten Cephalosporin-Endprodukts die Schutzgruppen wieder abgespalten werden.

Der Stand der Technik zur Herstellung von derartigen Verbindungen weist im wesentlichen drei Verfahren auf, die jedoch alle mit gewissen Nachteilen behaftet sind. So beschreibt die DE-OS 2,804.896 (TOYAMA) die Umsetzung von 7-Aminocephem(3)-4-carbonsäuren mit Lewis-Säuren bzw. mit Protonensäuren im Überschuß, wobei vorzugsweise Bortrifluorid verwendet wird. Es ist bekannt, daß derartige Reagenzien äußerst toxisch sind, Umweltprobleme verursachen und außerdem β-Lactame unter stark sauren Bedingungen im protischen Medium instabil sind.

Ein zweites Verfahren beschreibt die Austauschreaktion in 3-Stellung in wäßrigen Puffersystemen (US-PS 3,516.997). Dieses Verfahren gibt geringe Ausbeuten, da unter den verwendeten Bedingungen (7-ACA + heterocyclisches Thiol) Cephalosporine in Pufferlösungen zwischen pH 5 und 9 zu einem erheblichen Teil zersetzt und daher die Titelverbindungen in ungenügender Reinheit erhalten werden.

Ein drittes Verfahren beschreibt die Acylgruppen-Abspaltung der Seitenkette in 7-Position bei einem Cephalosporin, das schon den richtigen Substituenten in 3-Stellung trägt (z.B. US-PS 4,369.313). Zur Herstellung der in diesem Patent verwendeten Ausgangsmaterialien muß jedoch 7-ACA zunächst in 7-Stellung acyliert, dann der Austausch in 3-Stellung vorgenommen und zum Schluß die Seitenkette in Position 7 abgespalten werden. Gesamthaft gesehen ist diese Reaktionsfolge von der Ausbeute ab 7-ACA als unwirtschaftlich zu betrachten.

In der DE-A-3 316 798, Hoechst AG, ist u. a. ein Verfahren zur Herstellung von in 3-Stellung durch eine Base substituierte 7-Aminocephalosporansäure durch Zugabe der entsprechenden Base, gefolgt von Trimethyljodsilan zu der 7-Aminocephalosporansäure in einem Verdünnungsmittel und Erhitzen auf Rückfluss, oder durch Erhitzen der Base mit dem Trimethyljodsilan in einem Verdünnungsmittel auf Rückfluss und Zugabe der 7-Aminocephalosporansäure und weiteres Erhitzen auf Rückfluss beschrieben.

In EP-A-0 154 417, Yamanouchi Pharmaceutical Co. Ltd., die bezüglich der vorliegenden Anmeldung für die Vertragsstaaten Deutschland, Frankreich, Grossbritannien und Italien eine Veröffentlichung gemäss Art. 54(3) EPÜ darstellt, ist geoffenbart, dass 7-Aminocephalosporansäure erst in 3-Stellung jodiert und dann mit Hilfe eines Silylierungsmittels in 7- und 3-Stellung silyliert werden könne. Gemäss der Beschreibung wird angenommen, dass dabei 7-Trimethylamino-3-jodomethylcephem(3)-4-carbonsäuretrimethylsilylester als Zwischenprodukt entstehen soll.

Im vorliegenden Verfahren wird 7-ACA oder ein Derivat davon in ihr N,O-bissilyliertes Derivat überführt, hierauf mit einem Trialkyljodsilan zur 3-Jodmethylverbindung umgesetzt, durch Zugabe eines nucleophilen Reaktionspartners in das gewünschte 3-substituierte Derivat der N,O-bissilylierten 7-ACA überführt, dieses entweder entschützt oder mit einem Acylierungsmittel, das die gewünschte Acylgruppe in 7-Stellung einführen soll, versetzt und die Reaktion durch Entschützen zu Ende geführt.
Das volle Verfahren kann, wenn erwünscht, ohne Isolierung der einzelnen Zwischenprodukte durchgeführt werden.

Das Verfahren kann durch folgendes allgemeines Reaktionsschema beschrieben werden: 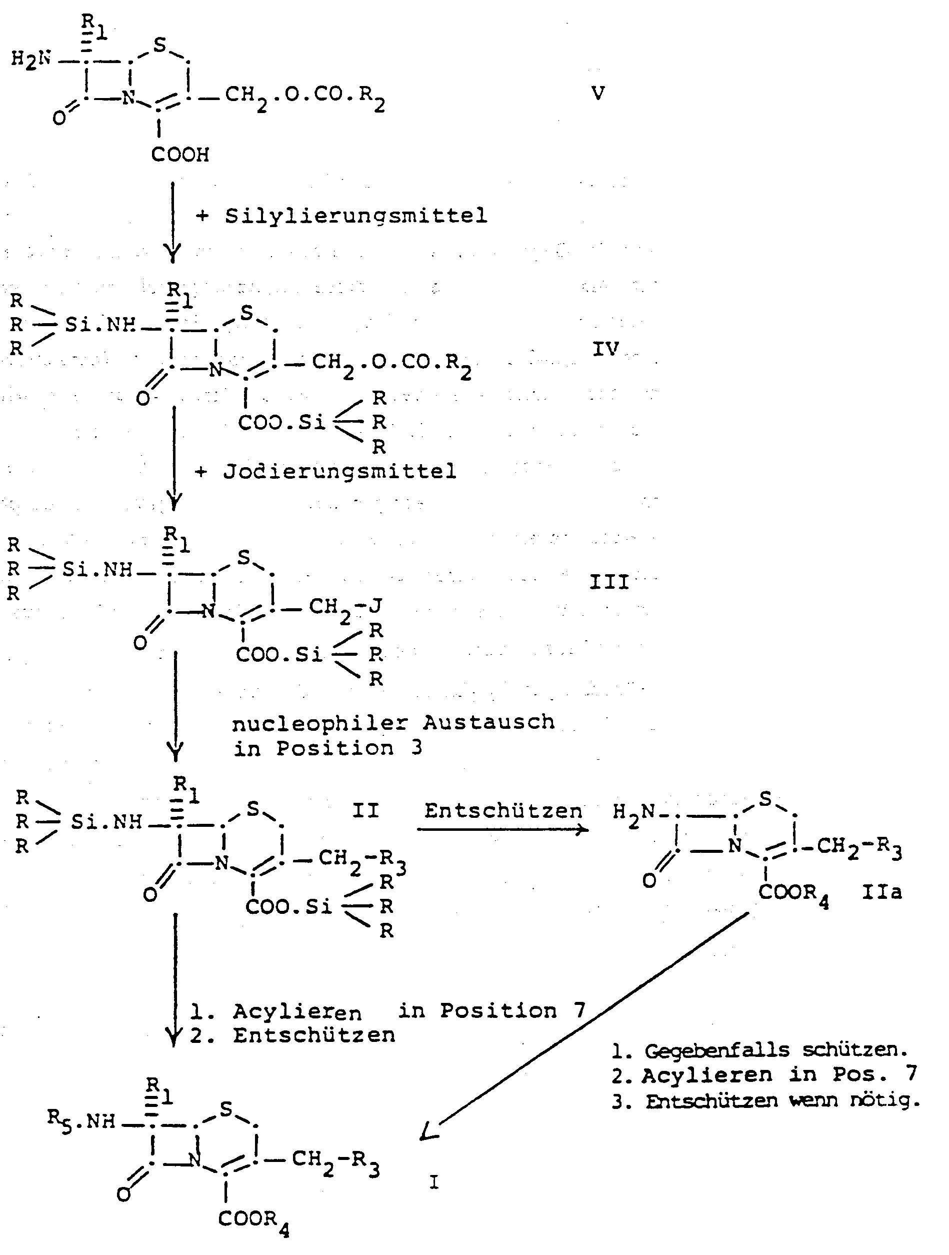
In den Formeln I bis V stehen R₁ für Wasserstoff oder die Methoxygruppe, R₂ für die Methyl- oder Aminogruppe, R für eine niedere Alkylgruppe und R₄ für Wasserstoff, Alkali oder eine negative Ladung bzw. OR₄ für Halogen. R₅ bedeutet eine in der Cephalosporinchemie übliche Acylgruppe. R₃ steht für den Rest eines Nukleophils. Die Erfindung betrifft die Stufe IV zu III.

Dieses Verfahren kann beispielsweise folgendermaßen ausgeführt werden:
Eine 7-Aminocephem(3)-4-carbonsäure der Formel V, vorzugsweise 7-ACA, wird in einem unter den Reaktionsbedingungen inerten Lösungsmittel suspendiert und in Gegenwart eines Überschusses an Silylierungsmittel rückflußerhitzt. Als Lösungsmittel für diese Silylierungsreaktion kommen halogenierte Kohlenwasserstoffe in Frage, wie beispielsweise Dichlormethan, Chloroform, 1,2-Dichlorethan, 1,1,2-Trichlorethan und Tetrachlorethylen, organische Nitrile, wie Acetonitril, Propionitril, Nitroalkane, wie Nitromethan oder Sulfone, wie Sulfolan. Die Silylierungsreaktion kann entweder bei hochsiedenden Lösungsmitteln durch Erhitzen auf die entsprechenden Temperaturen oder bei niedriger siedenden Lösungsmitteln durch eine spezielle Katalyse einer organischen Sauerstoffsäure, z.B. Quadratsäure, Trifluoressigsäure oder deren Gemische, durchgeführt werden. Als Silylierungsmittel bei dieser Reaktion können übliche Silylierungsmittel wie Hexamethyldisilazan, Trimethylchlorsilan, Bistrimethylsilylharnstoff, N-Trimethylsilyldialkylamine oder bis- oder monosilylierte Acetamide bzw. Gemische dieser Reagenzien, verwendet werden. Es ist bevorzugt, dass die Bissilylierung zu 100% verläuft, da geringe Mengen an Monosilylderivat die weitere Umsetzung erheblich stören können. Die Umsetzung der so erhaltenen Verbindungen der Formel IV wird beispielsweise so vorgenommen, dass zu dem erhaltenen Reaktionsgemisch 1,1 bis 1,5 Äquivalente Trialkyljodsilan, z.B. Trimethyljodilan zugegeben werden und die Reaktion bei Temperaturen von -20 bis +50°C, vorzugsweise bei 0 bis 45°C, durchgeführt wird. Es ist überraschend, dass eine Verbindung der Struktur von 3-Jodmethyl-N,O-bistrialkylsilyl-7-aminocephalosporansäure stabil ist. Bei einigen ähnlichen Strukturen, die in der Literatur beschrieben werden, erfolgt exzessive Selbstalkylierung im Stickstoff (DE-OS 3,212,900, YAMANOUCHI). Die Tatsache, dass die erfindungsgemässen N,O-Bis-trialkylsilyl-3-jodmethyl-7-aminocephalosporansäuren stabil sind, kann der sterischen Hinderung der Trialkylsilylgruppe am Stickstoff zugeschrieben werden. Es ist deshalb essentiell, reine Bissilylverbindung vorliegen zu haben, da das Vorliegen von Monosilylverbindungen zu exzessiver Zersetzung und Polymerisation führt.

Bistrimethylsilyl-3-jodmethyl-7-aminocephem(3)-4-carbonsäuren der Formel III können durch Entfernen des überschüssigen Lösungsmittels und Trialkyljodsilans im Vakuum in reiner Form isoliert und mittels des NMR-Spektrums charakterisiert werden.

Die Cephalosporinderivate der Formel III können leicht in 3-substituierte 7-Aminocephem(3)-4-carbonsäuren der Formel II bzw. IIa überführt werden, wobei die Verbindungen der Formel III nicht isoliert werden müssen, und zwar durch Umsetzung mit einem Nukleophil, wie z.B.
a) einem tertiären (Stickstoff) Heterocyclus (z.B. Pyridin, Chinolin) oder einem tertiären aliphatischen oder cycloaliphatischen Amin, z.B. N-Ethylpiperidin oder N-Methylpyrrolidin, oder
b) einem Thiolatanion eines mercaptosubstituierten Heterocyclus oder dem freien Mercaptoheterocyclus und einem Säurefänger, oder
c) einem, einen sekundären Stickstoff tragenden, Heterocyclus, und gegebenenfalls gleichzeitiger oder anschließender Entschützung.

Speziell kommen als solche Reaktionspartner für die Verbindungen der Formel III in Frage:
a) aromatische Stickstoffbasen wie Pyridin, Chinolin, die durch einen oder mehrere Substituenten zusätzlich substituiert sein können; aliphatische Trialkylamine, wobei Alkyl vorzugsweise die Bedeutung von C₁ - C₄ hat, und carbocyclische, gesättigte oder ungesättigte tertiäre Amine, z.B. N-Ethylpiperidin, N-Methylpyrrolidin, Chinuclidin u.a.m.,
b) Salze von mercaptosubstituierten Heterocyclen bzw. die freien Mercaptoverbindungen in Gegenwart von Säurefängern,
c) Heterocyclen, die einen sekundären, alkylierten Stickstoff haben, z.B. Triazole, Tetrazole, Pyrazole, Pyrrolidine, Imidazole.

Diese Umsetzungen mit obigen Reaktionspartnern können in den obgenannten Lösungsmitteln bei Temperaturen von -78 bis +70° C, vorzugsweise -50 bis +40° C, durchgeführt werden. Bei schwerer Löslichkeit eines Reaktionspartners in obgenannten Lösungsmitteln kann nach Bedarf ein absolut trockenes, aprotisches, polares Lösungsmittel, z.B. DMF, HMPT, zugesetzt werden.

Anschließend wird zur Herstellung der Verbindungen der Formel I im gleichen Reaktionsgemisch nach an sich bekannten Methoden die gewünschte Acylgruppe in Position 7 eingeführt und, falls vorhanden, die Silylgruppe abgespalten.

Für die Acylierung werden vorzugsweise Verbindungen der Formel II bzw. IIa in der Reaktionsmischung hergestellt, worin R₁ obige Bedeutung besitzt und R₃
a) eine aromatische Stickstoffbase der Formel 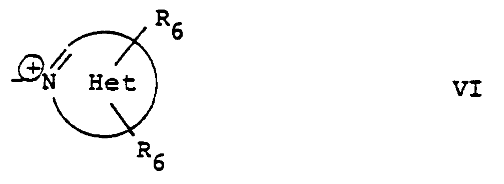 wobei Het Pyridin, Chinolin, Pyrimidin oder Thiazol und R₆ Carboxy, Carbamido, einen Sulfonsäurerest oder einen in der β-Lactamchemie üblichen Substituenten bedeuten, oder beide R₆ einen gegebenenfalls substituierten carbocyclischen Ring bilden, oder
b) eine aliphatische oder cycloaliphatische Stickstoffbase der Formel 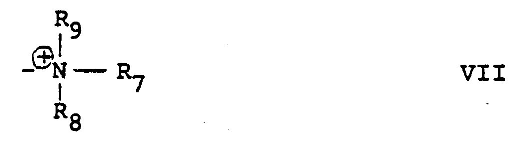 wobei die Substituenten R₇, R₈ und R₉ gleich oder verschieden sein können und jeweils eine niedere Alkyl- oder niedere Alkenylgruppe bedeuten oder R₇ mit R₈ und dem Stickstoffatom einen durch R₉ alkylsubstituierten fünf- oder sechsgliedrigen carbocyclischen gesättigten oder ungesättigten Ring bedeuten, wobei R₉ zusätzlich eine 1,3- oder 1,4-Alkylen- oder Vinylenbrücke darstellen kann, oder
c) einen gegebenenfalls substituierten heterocyclischen Thiolrest der Formel 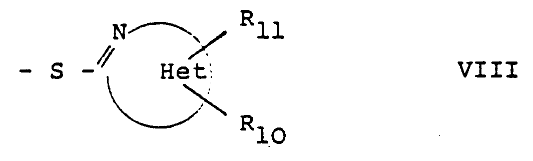 in dem Het einen fünf- oder sechsgliedrigen Heterocyclus bedeutet und R₁₀ und R₁₁ für Alkyl, Halogen oder Alkenyl steht, wobei ein Alkylrest wieder durch Sulfonsäuregruppen, Acylamino, Dialkylamino, Oxo oder Hydroxy substituiert sein kann, oder
d) einen Rest der Formel 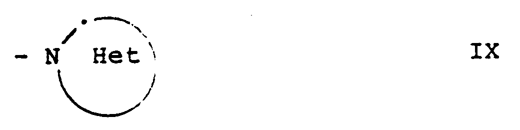 wobei Het Tetrazol, Triazol, Imidazol, Pyrrolidin oder Pyrazol, die durch eine niedere Alkylgruppe substituiert sein können, bedeutet. Diese Verbindungen können durch Zusatz einer Verbindung der Formeln 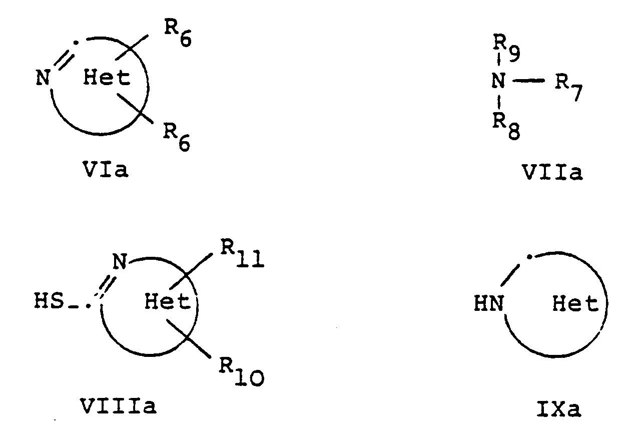 worin R₆ bis R₁₁ und Het obige Bedeutung besitzen, zum Reaktionsgemisch, das eine Verbindung der Formel II bzw. IIa enthält, erhalten werden.

In den nachfolgenden Beispielen, die die Erfindung erläutern sollen, ohne jedoch ihren Umfang einzuschränken, erfolgen alle Temperaturangaben in Celsiusgraden.

### Beispiel 1: 7-Trimethylsilylamino-3-jodomethylcephem(3)-4-carbonsäuretrimethylsilyester (Verbindung der Formel III)

### a) 7-Trimethylsilylamino-3-acetoxymethylcephem(3)-4-carbonsäuretrimethylsilylester (Formel IV):

Zu einer siedenden Suspension von 2,5 g 7-Aminocephalosporansäure, 1,75 ml Trichlorsilan und 7,65 ml Hexamethyidisilazan in 100 ml trockenem Chloroform gibt man 45 mg Quadratsäure und kocht das Gemisch unter Stickstoffatmosphäre über Nacht unter Rückfluß. Die klare Lösung wird dann unter Vakuum zur Trockene eingedampft, wobei der 7-Trimethylsilylaminocephalosporansäuretrimethylsilylester in fester Form erhalten wird. Durch die extreme Hydrolysierbarkeit kann keine Schmelzpunktsbestimmung durchgeführt werden, die Charakterisierung erfolgt NMR-spektroskopisch.
¹HNMR: 0.15 (9H, N-Si〈̶); 0.36 (s, 9H, -CO₂Si〈̶); 1.43 (d, J= 12 Hz, 1H, NH); 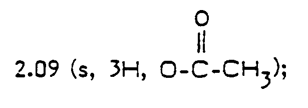
3.46 (AB, J = 18 Hz, 2H, C₂-H); 4.60 - 5.28 (m, 4H, CH₂-J, C₆-H, C₇-H).

### b) 7-Trimethylsilylamino-3-jodomethylcephem(3)-4-carbonsäuretrimethylsilylester (Formel III):

Die N,O-bissilylierte 7-ACA (wie unter a) beschrieben hergestellt) wird in 125 ml trockenem Dichlormethan gelöst, die Lösung auf 0° gekühlt und 1,87 ml Trimethyljodsilan zugetropft. Man läßt die Lösung auf Raumtemperatur kommen und rührt 2 Stunden nach. Nach Abdampfen des Lösungsmittels im Vakuum erhält man die Titelverbindung in fester Form, die wegen der großen Hydrolyseempfindlichkeit nur NMR-spektroskopisch charakterisiert wurde.
¹HNMR(CDCl₃): 0.125 (b, 9H, N-Si〈̶); 0.38 (s, 9H, -CO₂Si〈̶); 1.55 (d, J = 12 Hz, 1H, NH); 3.50 (AB, J = 18 Hz, 2H, C₂H); 4.39 (AB, J = 9 Hz, 2H, -CH₂-J); 4.58 (d, d, J = 12 Hz, 4,5 Hz, 1H, C₇-H); 4.71 (d, J = 4,5 Hz, 1H, C₆-H).

Aus dem 7-Trimethylsilylamino-3-jodomethylcephem(3)-4-carbonsäuretrimethylsilylester können beispielsweise folgende 3-substituierte Derivate der Formel II hergestellt werden:

### Beispiel 2: 7-Amino-3-pyridiniummethylcephem(3)-4-carbonsäure.Hydrojodid (Formel IIa):

Zu der Lösung der Titelverbindung des Beispiels 1 gibt man unter Eiskühlung 1,47 ml Pyridin und rührt die Lösung anschließend 2 Stunden bei Raumtemperatur. Durch Zugabe von 1,7 ml Methanol zur Lösung fällt man die rohe 7-Amino-3-pyridiniummethylcephem(3)-4-carbonsäure als Hydrojodid aus. Digerieren des Feststoffes in Wasser liefert die reine Titelverbindung. Fp: 145° (Zers.).
¹HNMR (D₂O, K₂CO₃): 3.38 (AB, J = 18 Hz, 2H, C₂-H); 4.80 (d, J = 5,8 Hz, 1H, C₇-H); 5.10 (d, J = 5,8 Hz, 1H, C₆-H); 5.43 (AB, J = 17 Hz, 2H, CH₂-N⁺〈̶); 8.0 - 8.2 (m, 2H, Pyridin 3H's); 8.41 - 8.63 (m, 1H, Pyridin-C₄-H); 8.93 (d, J = 6,3 Hz, 2H, Pyridin-2H's).

### Beispiel 3: 7-Amino-3-[5-(1,2,3-triazolyl)thiomethyl]cephem(3)-4-carbonsäure (Formel IIa):

Zu der Lösung der Titelverbindung des Beispiels 1 in Dichlormethan gibt man bei 0° 5 ml N,N-Dimethylformamid und 2,26 g 5-Mercapto-1,2,3-triazol.Na-salz und rührt die Lösung 2 Stunden bei Raumtemperatur weiter. Man gibt dann unter Eiskühlung 5 ml Methanol zu, wobei die Titelverbindung ausfällt. Man löst sie in 6 N HCl und stellt den pH mit konz. NaOH auf pH = 1, wobei die Titelverbindung wieder ausfällt. Zersetzungspunkt > 190°.
NMR (DMSO; CF₃CO₂H): 3.28 (AB, J = 18 Hz, 2H); 3.95 (AB, J = 12,6 Hz, 2H); 4.76 (d, J = 5,7 Hz, 1H); 4.97 (d, J = 5,7 Hz, 1H); 7.9 (s, 1H).

### Beispiel 4: 7-β-Amino-3-(2-dehydrochinuclidinium)methylcephem(3)-4-carboxylat.Dihydrochlorid (Formel IIa):

2,22 g 7-Aminocephalosporansäure werden wie in Beispiel 1 beschrieben zu 7-Trimethylsilylamino-3-jodomethylcephem(3)-4-carbonsäuretrimethylsilylester umgesetzt. Zu dieser Lösung in Dichlormethan gibt man unter Eiskühlung 1 g 1-Aza-bicyclo[2.2.2]oct-2-en und rührt dann 2 Stunden bei Raumtemperatur nach. Man fällt das Rohprodukt durch Zugabe von 2 ml gesättigter, etherischer HCl. Das Rohprodukt wird in verdünnter, wäßriger HCl gelöst und mit Isopropanol ausgefällt. Man erhält ein farbloses Pulver. Zersetzung ab 140°.
¹HNMR (D₂O): 1.70 - 2.50 (m, 4H); 3.0 - 5.2 (m); 3.76 und 4.10 (AB, J = 19 Hz, 2H); 5.34 (d, J = 6 Hz, 1H); 5.53 (d, J = 6 Hz, 1H); 6.70 - 7.3 (m, 2H).

### Beispiel 5: 7-β-Amino-3-(N-methylpyrrolidinium)methylcephem(3)-4-carboxylat.Hydrochlorid(Formel IIa):

10,88 g 7-Aminocephalosporansäure werden wie beschrieben zu 7-Trimethylsilylamino-3-jodomethylcephem(3)-4-carbonsäuretrimethylsilylester umgesetzt. Zur Lösung dieser Verbindung in 120 ml Dichlormethan gibt man bei -60° langsam 4,15 ml N-Methylpyrrolidin und rührt 90 Minuten bei dieser Temperatur. Das Reaktionsgemisch wird dann auf 100 ml Ethanol, das mit etwas HCl-Gas versetzt ist, gegossen. Anschließend stellt man den pH der Suspension auf 2,5. Nach Stehen über Nacht wird der Festkörper isoliert. Er wird in 20 ml Wasser suspendiert und der pH wird mit konzentrierter HCl auf 0 gestellt. Man gibt dann 5 g Aktivkohle zu, rührt 5 Minuten und filtriert vom Ungelösten und der Aktivkohle ab. Der pH der farblosen wäßrigen Lösung wird dann mit 5 N NaOH auf 2,5 gestellt und zur Lösung werden langsam 200 ml Aceton zugegeben. Nach zweistündigem Stehen im Kühlschrank wird der Niederschlag über eine Nutsche isoliert und mit Aceton und Ether gewaschen. Nach Trocknen im Trockenschrank erhält man ein farbloses Pulver. Zersetzungspunkt: 115°. Ausbeute: 5,6 g (42 % d. Th.).
¹HNMR (CF₃CO₂D+D₂O): 5.48 (1H, d, J = 5,2 Hz, C₇-H); 5.31 (1H, d, J = 5,2 Hz, C₆-H); 4.73 (2H, AB, J = 13 Hz; CH₂-N-); 4.1-3.47 (6H, m, C₂-H und N-methyl-pyrrolidin-H); 3.15 (3H, s, CH₃-N-); 2.37 (4H, b, N-methyl-pyrrolidin-H).

Verbindungen der Formel I können folgendermaßen erhalten werden:

### Beispiel 6: (6R,7R)-7-[2-(2-Amino-4-thiazolyl]-(Z)-2-[(1-diphenylmethoxy carbonyl-1-methylethoxy)imino]acetamido-3-(1-pyridiniummethyl)cephem(3)-4-carbonsäurechlorid.Hydrochlorid (Formel I):

Zu einer siedenden Suspension von 2,5 g 7-ACA, 1,75 ml Trichlorsilan und 7,65 ml Hexamethyldisilazan in 100 ml trockenem Chloroform gibt man 45 mg Quadratsäure und kocht das Gemisch unter Stickstoffatmosphäre über Nacht unter Rückfluß. Die klare Lösung wird dann unter Vakuum zur Trockne eingedampft, wobei der 7-Trimethylsilylaminocephalosporansäuretrimethylsilylester in fester Form ausfällt. Man lost ihn in 125 ml trockenem Dichlormethan, kühlt auf 0° ab und tropft 1,87 ml Jodtrimethylsilan zu. Man läßt die Lösung auf Raumtemperatur kommen und rührt 2 Stunden nach. Zum entstandenen 7-Trimethylsilylamino-3-jodomethylcephem(3)-4-carbonsäuretrimethylsilylester gibt man 1,47 ml trockenes Pyridin und rührt 2 Stunden bei Raumtemperatur. Anschließend gibt man zu dieser Lösung 5,41 g 2-(2-Amino-4-thiazolyl)-(Z)-2-[(1-diphenylmethoxycarbonyl-1-methylethoxy)imino]-thioessigsäure-S-benzothiazol-2-ylester und rührt das Gemisch 2 Tage bei Raumtemperatur. Das Reaktionsgemisch wird dann in eine 50° warme Lösung vor 4,5 ml konzentrierter Salzsäure in 500 ml Isopropanol eingetragen, wobei die Titelverbindung ausfällt. Sie wird abgesaugt und mit Isopropanol gewaschen. Nach Trocknung und Umkristallisation aus wäßriger HCl/Isopropanol erhält man die Titelverbindung als gelblich gefärbtes Produkt.
Fp.: Zersetzung ab 160°
¹H-NMR (DMSO-d₆): 9.84 (1H, d, -N-H, J = 8 Hz); 9.20, 8.67 und 8.23 (5H, Pyridinium-H); 7.28 (10H, Phenyl-H); 6.98 (1H, S, Thiazolyl-H); 6.79 (1H, S, CH-Ph₂); 5.95 (1H, dd, H₇,. J = 8 Hz, J = 5 Hz); 5.72 (2H, CH₂-Pyridin); 5.27 (1H, d, H₆, J = 5 Hz); 3.57 (2H, H₂ und H₂'); 1.63 (6H, -C(CH₃)₂).

### Beispiel 7: 7-[α-(Ethoxycarbonylmethoxy)imino-1H-pyrazol-3-yl]acetamido-3-[(1-methyl-1H-tetrazol-5-yl)thiomethyl]cephem(3)-4-carbonsäure.Ethanolsolvat (Formel I):

Zu einer Lösung von 3,82 g 7-Trimethylsilylamino-3-jodomethyl-3-cephem-4-carbonsäuretrimethylsilylester (hergestellt wie in Beispiel 1 oder 6 beschrieben) in 125 ml trockenem Dichlormethan gibt man 5 ml Dimethylformamid und 1,4 g 1-Methyl-5-mercapto-1,2,3,4-tetrazol.Natriumsalz und rührt die Lösung 2 Stunden bei Raumtemperatur. Anschließend gibt man 4,16 g kristallinen (Methoxycarbonylmethoximino)-1H-pyrazol-3-yl-essigsäure-mercaptobenzthiazolylester zu und rührt das Reaktionsgemisch über Nacht bei Raumtemperatur. Der Ansatz wird nun in 350 ml auf 55° vorgewärmtes Ethanol eingerührt und langsam auf 30° abgekühlt. Durch Animpfen mit der Titelverbindung fällt die Titelverbindung in kristalliner Form aus, sie wird abfiltriert und getrocknet.
Fp.: 119 - 121°.

### Beispiel 8: 7-β-(2-Thienylacetamido)-3-pyridiniummethylcephem(3)-4-carbonsäure.Nitrat (Formel I):

Zu einer Lösung von 3,82 g 7-Trimethylsilylamino-3-jodomethylcephem(3)-4-carbonsäuretrimethylsilylester (hergestellt wie in Beispiel 1 oder 6 beschrieben) in 125 ml trockenem Dichlormethan gibt man 1,47 ml trockenes Pyridin und rührt 2 Stunden bei Raumtemperatur. Anschließend gibt man zu dieser Lösung eine Lösung von 3,2 g Thienylessigsäure-5-mercaptobenzthiazolylester in 20 ml Dichlormethan (hergestellt in situ aus 1,56 g Thiophenessigsäure, 4,47 g Bis(benzothiazol-2-yl)disulfid und 3,53 g Triphenylphosphin in Dichlormethan) und rührt das Gemisch 30 Stunden bei Raumtemperatur. Durch Zugabe von 5 ml Methanol fällt man die rohe Titelverbindung als Hydrojodid. Diese wird in 5 % Bicarbonatlösung gelöst, von Unlöslichem abfiltriert, und durch Zugabe von verdünnter Salpetersäure kristallisiert die Titelverbindung bei einem pH von 1,8 als Nitrat aus.
Fp.: 151 - 152° (Zers.).

## Patentansprüche

1. Verfahren zur Herstellung eines Cephalosporanderivats der Formel III 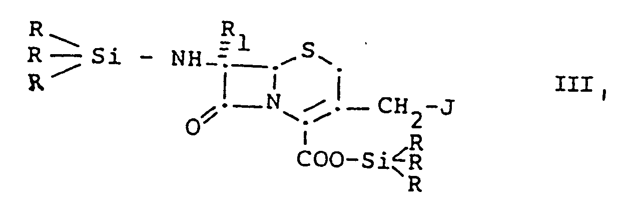 worin R für eine niedere Alkylgruppe und R₁ für Wasserstoff oder die Methoxygruppe stehen, dadurch gekennzeichnet, dass man eine Verbindung der Formel 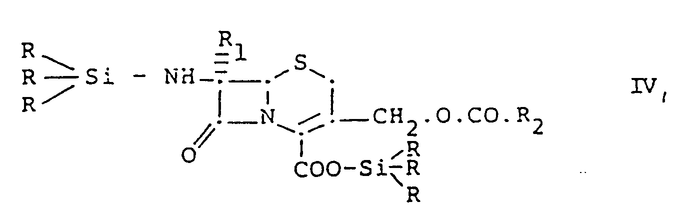 worin R und R₁ die oben angegebene Bedeutung haben und R₂ für die Methyl- oder die Aminogruppe steht, mit einem Trialkyljodsilan umsetzt.

2. Verfahren nach Anspruch 1, dadurch gekennzeichnet, dass das Trialkyljodsilan Trimethyljodsilan ist.

3. Verfahren nach einem der Ansprüche 1 oder 2, dadurch gekennzeichnet, dass man 1,1 bis 1,5 Äquivalente Trialkyljodsilan einsetzt.

## Claims

1. A process for the preparation of a cephalosporin derivative of formula 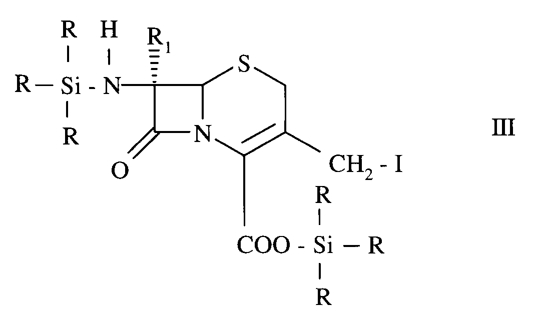 wherein R denotes a lower alkyl group and R₁ denotes hydrogen or the methoxy group, characterized in that a compound of formula 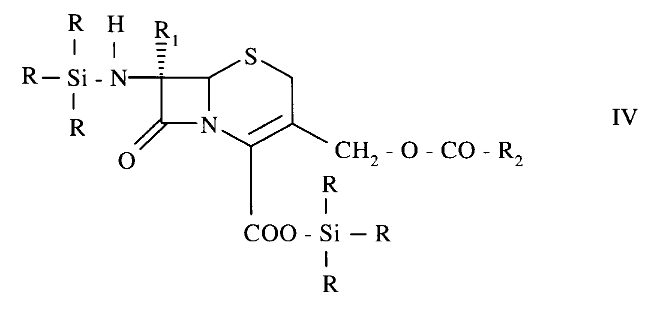 wherein R and R₁ are as defined above and R₂ denotes the methyl or the amine group is reacted with a trialkyliodosilane.

2. The process of claim 1, characterized in that the trialkyliodosilane is trimethyliodosilane.

3. The process according to anyone of claims 1 or 2 characterized in that 1.1 to 1.5 equivalents of trialkyliodosilane are used.

## Revendications

1. Procédé de préparation d'un dérivé de la céphalosporine de formule 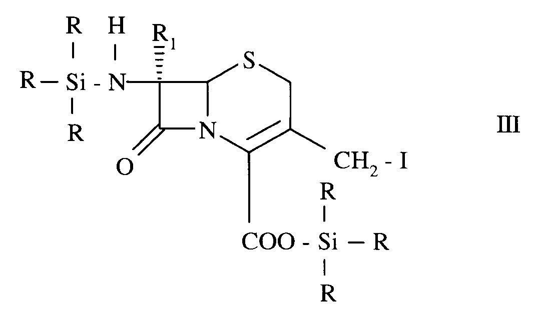 dans laquelle R signifie un groupe alkyle inférieur et R₁ signifie l'hydrogène ou un groupe méthoxy, caractérisé en ce qu'on fait réagir un composé de formule 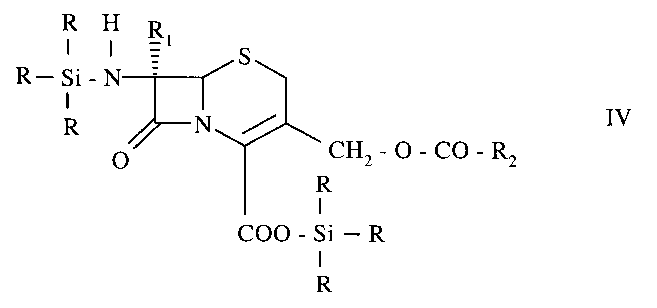 dans laquelle R et R₁ ont les significations données ci-dessus et R₂ signifie un groupe méthyle ou un groupe amino, avec un trialkyliodosilane.

2. Procédé selon la revendication 1, caractérisé en ce que le trialkyliodosilane est le triméthyliodosilane.

3. Procédé selon la revendication 1 ou 2, caractérisé en ce qu'on met en jeu de 1.1 à 1.5 équivalent de trialkyliodosilane.
